Europäisches Patentamt

European Patent Office

Office européen des brevets

(11) Publication number:

**0 346 866**
**A2**

## EUROPEAN PATENT APPLICATION

(21) Application number: 89110790.6

(51) Int. Cl.⁴: **A23L 1/304**

(22) Date of filing: 14.06.89

(30) Priority: 16.06.88 AT 1565/88

(43) Date of publication of application:
20.12.89 Bulletin 89/51

(84) Designated Contracting States:
AT BE CH DE ES FR GB GR IT LI LU NL SE

(71) Applicant: BIOCHEMIE GESELLSCHAFT M.B.H.

A-6250 Kundl(AT)

(72) Inventor: Helk, Walter
Buchengasse 133/4/24
A-1100 Vienna(AT)
Inventor: Zeisl, Erich
Wiesing 74 b
A-6200 Jenbach(AT)

(74) Representative: Kleine Deters, Johannes et al
Sandoz AG Patentabteilung
CH-4002 Basel(CH)

(54) Composition for the prophylaxis of osteoporosis.

(57) The invention concerns sweetener compositions supplemented with calcium.

EP 0 346 866 A2

## Composition for the prophylaxis of Osteoporosis

For the prevention of osteoporosis, the regular intake of calcium products over a prolonged period is indicated. This creates problems of patient compliance, however, since in general the patient is not conscious of any pain and the willingness to take tablets or other forms with the appearance of medicaments is low. Successful treatment with such forms therefore requires a significant change in the attitude of patients and it is clear that the longer the required period of administration, the less likely it is that regular intake will be maintained. For improvement of compliance, it would be advantageous to integrate calcium-intake into an existing daily routine and to avoid any noticable change in eating and drinking habits. The calcium product to be administered should therefore be contained in a product which is already a normal component of the patient's diet. One such component is sweetening compositions routinely used for drinks, such as coffee or tea.

The invention accordingly provides a sweetening composition. A sweetening composition in a form suitable for sweetening individual drinks including tea or coffee which is supplemented with a pharmaceutically acceptable, water-soluble calcium source in an amount appropriate to provide a recommended daily dosage of calcium for the prophylaxis of osteoporosis when the sweetening composition is consumed daily in sweetened drinks. The composition of the invention is in the first line a sweetener for addition to drinks and therefore should resemble as far as possible, and should preferably be visually undistinguishable from, the unsupplemented sweetening composition, and should be quickly and completely soluble. Obviously, the taste of the sweetening composition should not be adversely influenced by the calcium supplement.

Known calcium products do not meet these requirements. Many such products are in the form of effervescent tablets, as for example described in W. German DOS 3720510. This patent application describes a composition comprising a calcium salt, calcium carbonate and an organic polycarboxylic acid such as citric acid. On addition to water an effervescent solution results from the reaction of the calcium carbonate and citric acid with release of carbon dioxide. The resulting drink has an acidic taste. Such formulations obviously are not useful as sweetener's for addition to drinks such as coffee or tea.

Other known calcium preparations, such as tablets, ampoules, gels, capsules, as described for example in the Austria Codex, also do not meet the requirements of the compositions of the invention, even though the known oral forms may in some instances contain sweeteners. In particular, they do not meet the requirement of resemblence to a normal sweetener for drinks, the requirement of fast and complete solubility and/or the requirement of sweetening power and taste neutrality.

The unsupplemented sweetening composition for use in the compositions of the invention may comprise a sugar such as saccharose, any sugar substitute such as those conventionally used in foodstuffs or drinks, e.g. sorbite, xylite, fructose or glucose, or an artificial sweetener such as sodium saccharin, sodium cyclamate, acesulfam or aspartam.

The calcium product employed as supplement in the compositions of the invention may be any pharmaceutically acceptable, orally resorbed, calcium salt conventionally used in calcium products, preferably a taste-neutral, soluble calcium salt such as calcium gluconate, lactate lactogluconate, levulinate, glubionate, gluceptate or ascorbate.

The form of the sweetening composition of the invention is preferably as close as possible to, and preferably visually undistinguishable from, that of the unsupplemented sweetening composition. In case of sugar (saccharose) therefore the compositions of the invention may for example be in the form of powder (analagous to castor sugar), granulates (analogous to crystal sugar) or compressed forms (analogous to sugar lumps). Such forms may also be used in the case of sugar substitutes. In the case of artificial sweeteners, suitable forms include compressed tablets or solution concentrates.

The quantity of calcium salt in the compositions of the invention depends on the required daily dosage of calcium and the average daily intake of the sweetening composition. In general, for the prophylaxis of osteoporosis a daily dosage of 500 to 1000 mg of calcium is recommended in addition to the normal calcium intake. The daily intake of sweetener added to drinks such as coffee or tea varies but is usually from two to ten units. By "unit" is meant in the case of sugar a teaspoonful or lump which is approximately 5 g and in the case of other sweeteners that quantity which is equivalent thereto in sweetening power. Artificial sweetener units are generally in the form of small tablets, one tablet being equivalent in sweetening power to one teaspoonful or lump or sugar, or concentration of which a measured quantity is likewise equivalent. Therefore the preferred calcium content per unit of sweetening composition is from 50 to 500 mg. The weight ratio of calcium to sweetener can be calculated accordingly. Thus, for example a unit (e.g. lump or teaspoonful) of sugar (saccharose) weights approximately 5 g. Therefore sugar compositions according to the invention contain preferably 10 to 100 mg of calcium per gram of sugar. The corresponding weight ratio for other sweeteners can be calculated similarly.

The calcium content values discussed above correspond to the quantity of ionisable calcium in the particular salt employed. The quantity of salt to be used to provide those quantities of ionisable calcium can be calculated accordingly.

The following Examples illustrate the invention.

## Example 1. Saccharose Powder

387.9 kg of powdered saccharose (castor sugar) is homogenously mixed with 112.1 kg of powdered calcium gluconate (monohydrate) (corresponding to 100 mg of ionisable calcium per 5 g of saccharose). The mixture is packed in 5 g sachets.

## Example 2. Saccharose Granulate

345.9 kg of crystalline saccharose (crystal sugar) are mixed with 154.1 kg of calcium lactate (pentahydrate) (corresponds to 200 mg of ionisable calcium per 5 g of mixture). The mixture is moistered with 7.5 kg of water, granulated and dried at 50°C.

## Example 3. Compound Saccharose (sugar lumps)

436.7 kg of crystalline saccharose are mixed with 38.3 kg of calcium laevulinate (dihydrate). The mixture is moistered with a solution of 25 kg of polyethyleneglycol 6000 in 30 kg of water and is then granulated and dried at 50°C. The granulate is sieved and compressed in a tablet press into lumps of 5 g each. Each lump contains 50 mg of ionisable calcium.

## Example 4. Sugar Substitute Granulate

387.9 kg of sorbite is mixed with 112.1 kg of calcium gluconate (monohydrate). The mixture is moistened with 7 kg of water, granulated and dried at 40°C. 5 g of the granulate contains 100 mg of ionisable calcium.

## Example 5. Sweetener Tablets

1 kg of sodium saccharin is mixed with 77 kg of calcium lactate (pentahydrate). The mixture is moistered with a solution of 7 kg of polyethylene glycol 6000 in 15 kg of water, granulated and dried at 50°C. The granulate is compressed into 850 mg tablets in a tablet press. Each tablet contains approximately 140 mg of ionisable calcium.

## Example 6. Sweetener Concentrate

1.2 kg of Sodium cyclamate, 0.12 kg of sodium saccharin, 0.25 kg of sorbic acid and 22.42 kg of calcium gluconate (monohydrate) are dissolved in 100 l of water. The solution is filtered until it is clear. 5 ml (which is equivalent to 1 teaspoonful) contains 100 mg of ionisable calcium and corresponds in sweetening power to approximately are teaspoonful of sugar (saccharose).

## Example 7. Sweetener Concentrate

11.2 kg calcium gluceptate, 6.1 kg clacium lactogluconate (dihydrate), 9.2 kg of calcium gluconate (monohydrate), 0.09 kg of methyl parehydroxbenzoate, 0.01 kg of propyl para-hydroxbenzoate, 2.4 kg of sodium clyclamate and 0.24 kg of sodium saccharin are dissolved in 94.4 l of water. The resulting solution is filtered until it is clear. 2 ml of this solution contains 50 mg of ionisable calcium and possesses a sweetening power approximately equivalent to that of 5 g of sugar (saccharose).

## Claims

1. A sweetening composition in a form suitable for sweetening individual drinks including tea or coffee which is supplemented with a pharmaceutically acceptable, water-soluble calcium source in an amount appropriate to provide a recommended daily dosage of calcium for the prophylaxis of osteoporosis when the sweetening composition is consumed daily in sweetened drinks.

2. A sweetening composition according to claim 1, which contains 50 to 500 mg of ionisable calcium per unit of sweetening composition.

3. A sweetening composition according to any of the preceding claims, in which the calcium source is neutral-tasting.

4. A sweetening composition according to any of the preceding claims which contains no additional flavouring agent.

5. A sweetening composition according to any of the preceding claims which is visually undistinguishable from the unsupplemented sweetening composition.

6. A sweetening composition according to any of the preceding claims in which the sweetening component is a sugar.

7. A sweetening composition according to claim 6 in which the sugar is saccharose.

8. A sweetening composition according to any of claims 1 to 5, in which the sweetening component is sorbite, xylite, fructose or glucose.

9. A sweetening composition according to any of claims 1 to 5, in which the sweetening component is sodium saccharin, sodium cyclamate, acesulfam or aspartam.

10. A sweetening composition according to any of the preceding claims in which the calcium source is calcium gluconate, calcium lactogluconate, calcium glubionate, calcium levulinate, calcium gluceptate or calcium ascorbate.